# EUROPÄISCHE PATENTSCHRIFT

(11) **EP 3 287 780 B1**
(45) Veröffentlichungstag und Bekanntmachung des Hinweises auf die Patenterteilung: **04.11.2020**
(21) Anmeldenummer: 17194930.8
(22) Anmeldetag: 08.12.2015
(51) Int. Cl.: G01N 31/12, G01N 33/18

(54) **ANALYSEANORDNUNG ZUR WASSER- UND ABWASSERANALYSE**
ANALYSIS SYSTEM FOR ANALYZING WATER AND WASTEWATER
SYSTÈME POUR L'ANALYSE D'EAU ET D'EAUX USÉES

(30) Priorität: 08.12.2014 DE 102014118138
(43) Veröffentlichungstag der Anmeldung: 28.02.2018
(62) Teilanmeldung aus: 15834790.6
(73) Patentinhaber: LAR Process Analysers AG, 12057 Berlin (DE)
(72) Erfinder: ARTS, Werner, 10825 Berlin (DE); GENTHE, Wolfgang, 14059 Berlin (DE)
(74) Vertreter: Meissner Bolte Partnerschaft mbB

(56) Entgegenhaltungen:
- EP-A1- 2 662 690
- DE-A1- 2 261 456
- US-A- 3 560 156
- US-A- 4 074 973

## Beschreibung

Die Erfindung betrifft eine Analyseanordnung zur Wasser- und Abwasseranalyse, die ein Analysegerät mit einem Gerätegehäuse und einem Injektionsport zur Einführung einer Probe in das Gerät sowie eine Injektionsspritze umfasst.

Derartige Analyseanordnungen sind an sich bekannt und werden auch von der Anmelderin hergestellt und angeboten.

US 3 560 156 A offenbart eine Analyseanordung mit den Merkmalen des Oberbegriffs von Anspruch 1.

Die Druckschriften US 4 074 973 A, EP 2 662 690 A1 sowie DE 20 2061 456 A1 offenbaren jeweils eine Analyseanordung mit einem zylindrischen Reaktionsgefäß und steuerbaren Mitteln zur Festigung von Druck und Durchflussmenge eines Trägergases, mit dem eine Probe dem Reaktionsgefäß zugeführt wird.

Der Erfindung liegt die Aufgabe zu Grunde, eine verbesserte Analyseanordnung dieser Art bereitzustellen, die insbesondere verlässlich genaue und exakt reproduzierbare Analyseergebnisse liefern kann.

Diese Aufgabe gelöst durch ein Analysegerät mit den Merkmalen des Anspruchs 1.

Erfindungsgemäß hat das Analysegerät einen Injektionsport, der mit einem zylindrischen Reaktionsgefäß zum thermischen Aufschluss des zu analysierenden Stoffes verbunden ist, und der weist Führungsmittel zum Führen einer Injektionsspritze in eine vorbestimmte Einspritzposition aufweist.

Die Erfindung schließt den Gedanken ein, dass mit dem Reaktionsgefäß eingangsseitig eine Trägergaszuführung verbunden ist, die steuerbare Mittel zur Festlegung von Druck und Durchflussmenge eines dem Reaktionsgefäß zuzuführenden Trägergases aufweist. Neben einer in ihrer Menge präzise bemessenen und im Ort und der Richtung des Ausstoßens der Probe in das Reaktionsgefäß präzise vorbestimmten Probenzuführung ist für exakte und reproduzierbare Analyseergebnisse auch eine genaue und reproduzierbare Steuerung des Trägergasstromes zum Transport der Probe zu einer Nachweiseinrichtung von großer Bedeutung. Es hat sich gezeigt, dass eine einfache Durchflussbegrenzung, etwa mittels eines Reduzierventils, den Genauigkeitsanforderungen einer für den Laboreinsatz geeigneten Analyseanordnung nicht genügt.

Insbesondere ist hierzu vorgesehen, dass die Trägergaszuführung einen ersten Zweig (Teilstrom) zur Zuführung von Stickstoff und einen zweiten Zweig (Teilstrom) zur Zuführung von Sauerstoff umfasst, wobei im ersten Zweig ein erster Druckregler und Durchflussregler zur Druckregelung bzw. Durchflussregelung von zugeführtem Stickstoff und im zweiten Zweig ein zweiter Druckregler und Durchflussregler zur Druckregelung bzw. Durchflussregelung von mit dem Stickstoff zu vermischendem Sauerstoff vorgesehen sind. Hierbei sind insbesondere die ersten und zweiten Druck- und Durchflussregler derart ausgebildet und abgestimmt, dass bei der Vermischung des Sauerstoffs mit dem Stickstoff eine Verdünnung um einen Faktor von mindestens 10, speziell mindestens 100 und noch spezieller von etwa 1000, realisierbar ist. Es versteht sich, dass zur Realisierung derart niedriger Verdünnungen mit hinreichender Genauigkeit Präzisionsdurchflussregler einzusetzen sind.

Gemäß der oben erwähnten Erfindung basiert diese auf der Überlegung, in Abhängigkeit von bestimmten Merkmalen des Analyseergebnisses eine Nachbearbeitung bzw. Beeinflussung des konkreten Auswertungsmodus zuzulassen. Die Erfinder haben festgestellt, dass bei der Bestimmung relevanter Bestandteile von Wasser- bzw. Abwasserproben die konkrete Probenzusammensetzung wie auch Eigenheiten des Ablaufs der Analyse die Signalform eines zeitabhängigen Nachweissignals erheblich beeinflussen können und dass sich hieraus die Zweckmäßigkeit einer "manuellen" Beeinflussung des Auswertungsvorganges ableitet.

Da speziell der zeitliche Signalverlauf bei zeitabhängig gemessenen Sauerstoffkonzentration und weiterhin Kohlendioxid-, Stickstoff- oder Phosphorkonzentrationen des Wassers oder Abwassers sehr unterschiedlich sind, lassen sich mit einer fest eingestellten Integrationszeit nur bedingt brauchbare und vergleichbare Analyseergebnisse erreichen. Es ist daher bevorzugt vorgesehen, dass die Nachbearbeitungseinrichtung zur manuellen Nach-Einstellung einer Integrationszeit zum Aufintegrieren der jeweils zeitabhängig erfassten Konzentrationswerte ausgebildet ist.

Vorteile und Zweckmäßigkeiten der Erfindung ergeben sich im Übrigen aus der nachfolgenden kurzen Beschreibung eines Ausführungsbeispiels anhand der Figuren. Von diesen zeigen:
Fig. 1 eine schematische Darstellung wesentlicher Gerätekomponenten eines Analysegerätes gemäß einem Ausführungsbeispiel,
Fig. 2 eine perspektivische Darstellung jenes Analysegerätes,
Fig. 3 eine schematische Längsschnittdarstellung einer Injektionsspritze mit in den Injektionsport eingeführter Injektionsnadel gemäß einer beispielhaften Ausführungsform der Analyseanordnung und
Fig. 4a und 4b grafische Darstellungen zur Illustration eines Aspekts der Erfindung.

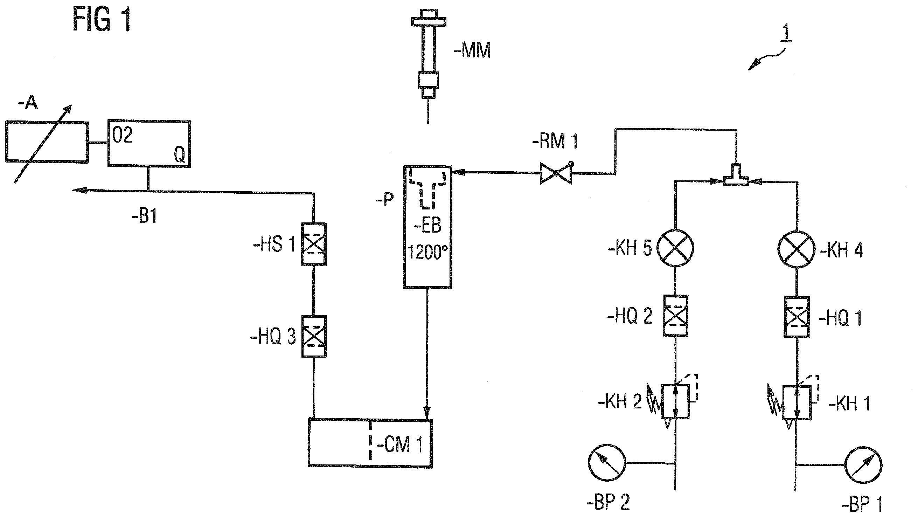

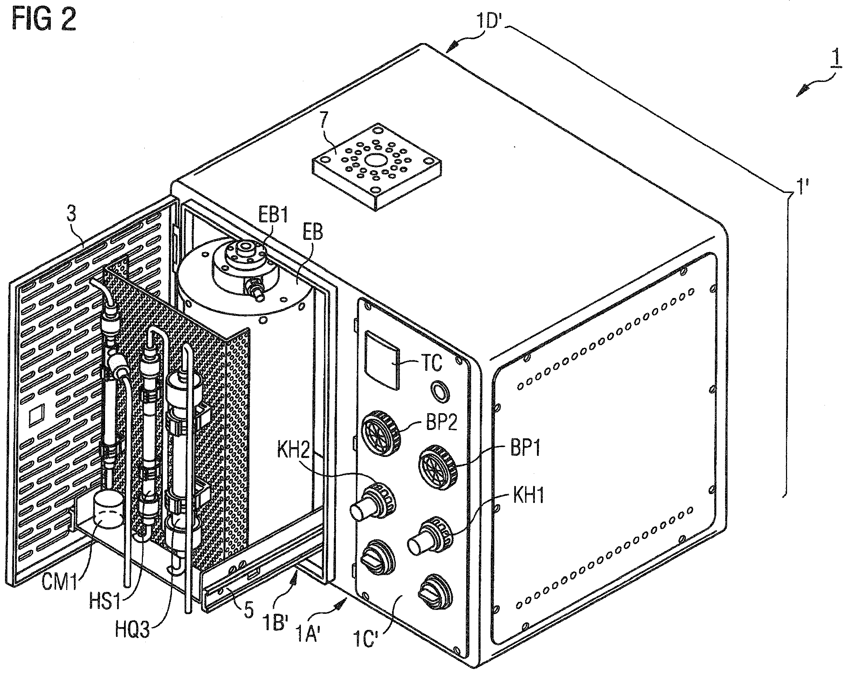

Fig. 1 und 2 zeigen schematisch wesentliche Teile eines Analysegerätes 1 zur Bestimmung des chemischen Sauerstoffbedarfs (CSB oder COD = chemical oxygen demand) von Wasser oder Abwasser. Der grundsätzliche Aufbau derartiger Geräte und deren Funktionsweise sind dem Fachmann bekannt und werden daher hier nicht näher beschrieben, zumal es hierauf zum Verständnis der vorliegenden Erfindung nicht ankommt.

Das Analysegerät 1 umfasst ein thermisches Reaktionsgefäß bzw. einen Ofen EB, in den mittels einer Injektionsspritze MM über einen an der Ofen-Oberseite angeordneten Injektionsport P eine Wasserprobe eingespritzt werden kann und in dem diese Probe thermisch aufgeschlossen wird. Dem Ofen wird über ein Rückschlagventil RM1 ein Trägergasstrom zugeführt, welcher aus Luft und Stickstoff zusammengesetzt wird. Der Trägergasstrom wird mittels eines Luft-Druckreglers KH1, eines Stickstoff-Druckreglers KH2, eines Luft-Durchflussreglers KH4 und eines Stickstoff-Durchflussreglers KH5 gesteuert und mittels eines ersten und zweiten Feinfilters HQ1, HQ2 eingangsseitig des Ofens gefiltert. Eingangsseitig des Ofens sind auch eine Luft-Druckanzeige BP1 und eine Stickstoff-Druckanzeige BP2 vorgesehen.

Ausgangsseitig des Ofens gelangt der Gasstrom zunächst in ein Kondensatgefäß CM1, und der nicht kondensierte Anteil durchläuft anschließend ein Quarzwollefilter HQ3 und eine Säurefalle HS1, bevor er zum Sauerstoffdetektor B1 gelangt, welcher schließlich einen (elektrischen) Messwert an eine einstellbare Auswertungseinrichtung A ausgibt, bei der insbesondere eine Integrationszeit zur Aufintegration eines zeitabhängig erfassten Sauerstoff-Nachweissignals vorgesehen ist; siehe dazu weiter unten. Das erfindungsgemäße Analysegerät umfasst auch einen Kohlendioxid- und/oder einen Stickstoff- und/oder einen Phosphor-Detektor welcher jedoch hier nicht näher beschrieben wird.

Fig. 2 zeigt das Analysegerät 1 in perspektivischer Darstellung in einem Zustand, in dem das Gerätegehäuse 1' teilweise geöffnet ist und ein Teil der Gerätekomponenten aus dem Gehäuse herausgezogen ist. Das Gerätegehäuse hat im Wesentlichen die Gestalt eines quadratischen Prismas, und in der Gerätefront 1A' ist eine Öffnung 1B' vorgesehen, die durch eine an der linken Seitenkante der Gerätefront angeschlagene, perforierte Tür 3 zu verschließen ist.

Ein in seinen Abmessungen an die Öffnung 1B' angepasster Schlitten 5, auf dem der Ofen EB, das Kondensatgefäß CM1, das Quarzwollefilter HQ3 und die Säurefalle HS1 angeordnet sind, ist so weit aus dem Gehäuse herausziehbar, dass die genannten Komponenten frei zugänglich werden. Im zurückgeschobenen Zustand des Schlittens 5 wird das Gerätegehäuse 1' mit der Tür 3 verschlossen.

Auf der rechten Seite der Gerätefront 1A' befindet sich ein Bedienfeld 1C', auf dem mehrere Bedien- und Anzeigeelemente angeordnet sind, darunter eine Temperaturanzeige /-steuerung TC und die Einstellregler KH1 und KH2 für den Luft- bzw. Stickstoffdruck und die zugehörigen Anzeigeelemente BP1 und BP1.

Auf der Geräteoberseite 1D' befindet sich der Injektionsport P, dessen Aufbau und Abmessungen an diejenigen der in Fig. 1 gezeigten Injektionsspritze MM angepasst sind und der im Geräteinneren mit einem entsprechenden Einspritzventil EB1 des Ofens EB kommuniziert, wenn der Ofen sich in seiner normalen Gebrauchslage innerhalb des Gerätegehäuses befindet.

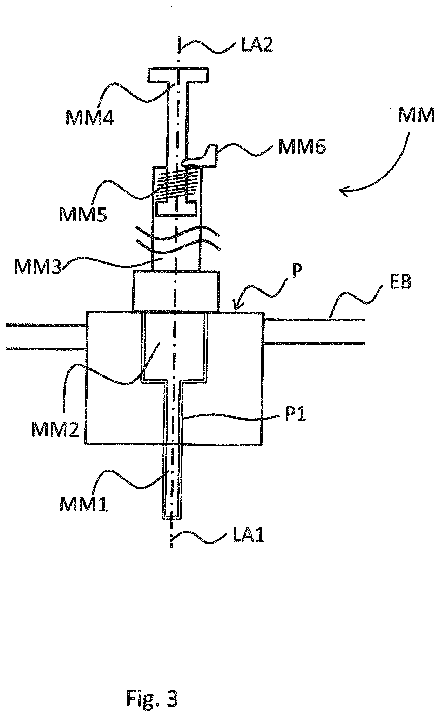

Fig. 3 zeigt skizzenartig in einer Längsschnittdarstellung den grundsätzlichen Aufbau und die aufeinander abgestimmte geometrische Konfiguration der Injektionsspritze MM und des Injektionsports P des Ofens EB der Analyseanordnung. Der Injektionsport P umfasst eine in ihrem Längsverlauf im Wesentlichen zylindrisch ausgebildete Führungshülse P1, deren Durchmesser und Länge auf die entsprechenden Abmessungen einer Injektionsnadel MM1 der Injektionsspritze MM abgestimmt sind und deren Längsachse mit einer Längsachse LA1 des in seiner Grundform zylindrisch ausgeführten Ofens EB zusammenfällt. An der Oberseite des Injektionsports P ist eine Bohrung P2 mit vergrößertem Durchmesser vorgesehen, deren Abmessungen auf diejenigen eines Nadel-Ansatzes MM2 der Injektionsspritze angepasst sind und deren untere Stirnfläche beim Einführen der Injektionsspritze als Anschlag zur Tiefenbegrenzung wirkt. Mit diesem Anschlag wird eine exakt vorbestimmte Position des rechtwinklig zur Nadel-Längsachse LA2 der Injektionsspritze abgeschnittenen Nadelendes im Ofen EB und somit ein exakt vorbestimmter Einspritzpunkt gewährleistet.

Im Spritzen-Reservoir MM3 ist ein Spritzenkolben MM4 längsverschieblich gelagert, dessen freies Ende in üblicher Weise zum manuellen Aufziehen einer Probe ausgestaltet ist. Am oberen Ende des Spritzenreservoirs ist in diesem eine Druckfeder MM5 eingebettet, deren oberes Ende sich gegen die obere Stirnwand des Spritzenreservoirs abstützt und deren unteres Ende auf das Ende des Spritzenkolbens MM4 wirkt. Nach dem Aufziehen der Spritze wird mittels eines Verriegelungshebels MM6 der Spritzenkolben mit gespannter Feder MM5 arretiert. Nach Lösen der Verriegelung MM6 wird der Spritzenkolben MM4 durch die Kraft der Druckfeder MM5 nach unten gedrückt und die im Spritzenreservoir MM3 enthaltene Probe in einem vorbestimmten Zeitintervall bzw. mit vorbestimmter Austraggeschwindigkeit in den Ofen eingespritzt.

Dieser Austrag der vorbestimmten Probenmenge mit exakt vorbestimmter Geschwindigkeit bzw. in einem exakt definierten Zeitintervall ist für reproduzierbare Analyseergebnisse ebenso wichtig wie die exakte Einspritzposition- und Richtung, die durch die spezielle Gestaltung der Injektionsnadel und des Injektionsports gesichert werden.

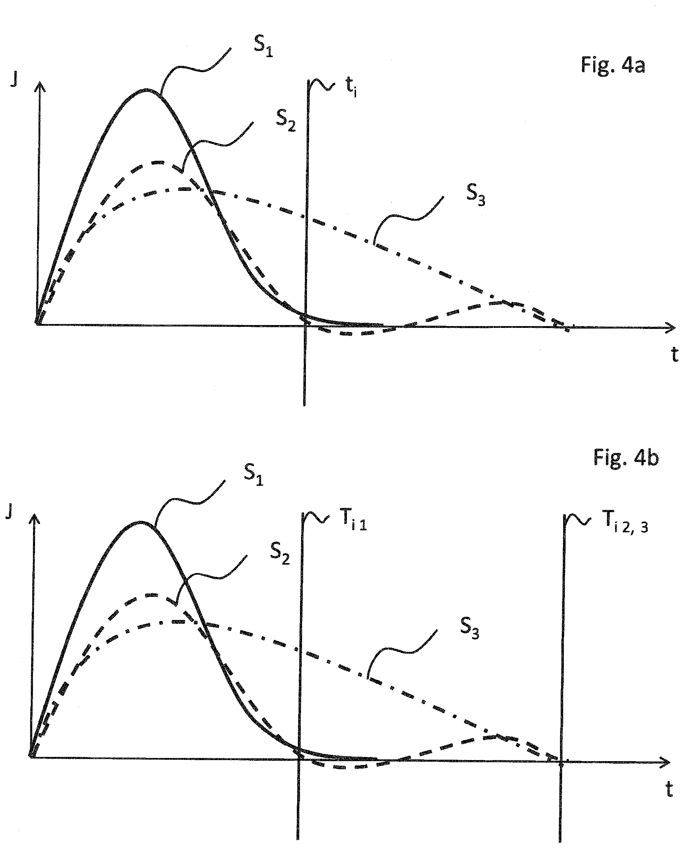

Fig. 4a und 4b illustrieren die Wirkung einer einstellbaren Integrationszeit der Nachbearbeitungseinrichtung A zur Verarbeitung eines zeitabhängig aufgenommenen Sauerstoff-Messsignals. Fig. 4a zeigt hierbei drei verschiedene Kurvenformen mit (gemäß dem Stand der Technik) fest eingestellter Integrationszeit tᵢ. Es ist erkennbar, dass hier nur die Aufintegration des mit der durchgezogenen Linie dargestellten Messsignals S₁ zu einem korrekten Ergebnis führt, während die eingestellte Integrationszeit für die Messsignale S₂ und S₃ offensichtlich zu kurz ist und wesentliche Signalanteile nicht erfasst werden. Abhilfe schafft hier das in Fig. 4b gezeigte Einstellen einer längeren Integrationszeit t_{i2, 3} für die Signale S₂ und S₃, die der Bediener der Analyseanordnung in Abhängigkeit der festgestellten Signalkurvenform an der Nachbearbeitungseinrichtung A vornehmen kann.

## Patentansprüche

1. Analyseanordnung zur Wasser- und Abwasseranalyse, mit einem zylindrischen Reaktionsgefäß zum thermischen Aufschluss des zu analysierenden Stoffes, wobei mit dem Reaktionsgefäß eingangsseitig ein Injektionsport, der Führungsmittel zum Führen einer Injektionsspritze für den zu analysierenden Stoff aufweist, und eine Trägergaszuführung verbunden ist, die steuerbare Mittel zur Festlegung von Druck und Durchflussmenge eines dem Reaktionsgefäß zuzuführenden Trägergases aufweist, wobei die Trägergaszuführung einen ersten Zweig zur Zuführung von Stickstoff und einen zweiten Zweig zur Zuführung von Sauerstoff umfasst, wobei im ersten Zweig ein erster Druckregler und Durchflussregler zur Druckregelung bzw. Durchflussregelung von zugeführtem Stickstoff und im zweiten Zweig ein zweiter Druckregler und Durchflussregler zur Druckregelung bzw. Durchflussregelung von mit dem Stickstoff zu vermischendem Sauerstoff vorgesehen sind, und wobei die Analyseanordnung weiter eine Nachweis- und Auswertungseinrichtung zum Nachweis eines vorbestimmten Bestandteils des zu analysierenden Stoffes aufweist,
**dadurch gekennzeichnet, dass**
die Nachweis- und Auswertungseinrichtung weiterhin eine einstellbare Nachbearbeitungseinrichtung zur manuell gesteuerten Nachbearbeitung des Nachweisergebnisses in Abhängigkeit einer festgestellten Signalkurvenform eines zeitabhängig aufgenommenen Messsignals des zu analysierenden Stoffes aufweist,
und dass die Nachweiseinrichtung einen Sauerstoff- und weiterhin einen Kohlendioxid- und/oder einen Stickstoff- und/oder einen Phosphor-Detektor zum zeitabhängigen Nachweis der Sauerstoffkonzentration und weiterhin der Kohlendioxid-, Stickstoff- und/oder Phosphorkonzentration in dem das Reaktionsgefäß verlassenden Trägergasstrom aufweist und die Nachbearbeitungseinrichtung zur manuellen Nach-Einstellung einer Integrationszeit zum Aufintegrieren der jeweils zeitabhängig erfassten Konzentrationswerte des Sauerstoffs und Kohlendioxids und/oder Stickstoffs und/oder Phosphors ausgebildet ist.

## Claims

1. Analyzing device for water- and waste water analysis, comprising a cylindrical reaction vessel for thermal disintegration of the substance to be analyzed,
wherein with the inlet side of the reaction vessel an injection port, which comprises guide means for guiding an injection syringe for the substance to be analyzed, and a carrier gas supply, which comprises controllable means for determining pressure and flow rate of a carrier gas to be supplied to the reaction vessel, are connected,
the carrier gas supply comprising a first branch for supplying nitrogen and a second branch for supplying oxygen,
wherein in the first branch a first pressure controller and flow controller for pressure control or flow control of supplied nitrogen and in the second branch a second pressure controller and flow controller for pressure control or flow control of oxygen to be mixed with the nitrogen are provided, and
wherein the analyzing device further comprises a detection and evaluation device for detecting a predetermined component of the substance to be analyzed,
**characterized in that**
the detection and evaluation device further comprises an adjustable post-processing device for manually controlled post-processing of the detection result in dependence of a determined signal waveform of a time-dependent recorded measurement signal of the substance to be analyzed,
The detection device comprises an oxygen detector and further a carbon dioxide and/or a nitrogen and/or a phosphorus detector for the time-dependent detection of oxygen concentration and further of the carbon dioxide, nitrogen and/or phosphorus concentration in the carrier gas stream exiting the reaction vessel, and
the post-processing device is configured for a manual re-adjustment of an integration time for the integration of the respective time-dependent concentration values of oxygen and carbon dioxide and/or nitrogen and/or phosphorus detected.

## Revendications

1. Système d'analyse permettant d'analyser l'eau et des eaux usées, pourvu d'un récipient de réaction cylindrique destiné à réaliser un traitement thermique de la matière à analyser, l'entrée dudit récipient de réaction étant reliée à un port d'injection comportant des moyens de guidage permettant de guider une seringue d'injection et à une arrivée de gaz vecteur comportant des moyens réglable permettant de définir la pression et le débit d'un gaz vecteur devant être introduit dans le récipient de réaction, ladite arrivée de gaz vecteur comprenant une première branche destinée à l'alimentation en azote et une deuxième branche destinée à l'alimentation en oxygène, un premier régulateur de pression et régulateur de débit étant disposé dans la première branche afin régler, respectivement, la pression et le débit de l'azote introduit, et un deuxième régulateur de pression et régulateur de débit étant disposé dans la deuxième branche afin régler, respectivement, la pression et le débit de l'oxygène destiné à être mélangé à l'azote, et le système d'analyse comportant en outre un dispositif de détection et d'évaluation permettant de détecter un constituant prédéfini de la matière à analyser,
**caractérisé en ce que**
ledit dispositif de détection et d'évaluation comporte en outre un dispositif de traitement ultérieur réglable permettant de soumettre le résultat de détection à un traitement ultérieur commandé manuellement, en fonction de la forme de courbe de signal telle qu'elle a été constatée, au cours du temps, pour un signal de mesure relatif à la matière à analyser,
et que le dispositif de détection comporte un détecteur d'oxygène et, en outre, un détecteur de dioxyde de carbone et/ou un détecteur d'azote et/ou un détecteur de phosphore, afin de détecter, au cours du temps, la concentration d'oxygène et, en outre, la concentration de dioxyde de carbone, d'azote et/ou de phosphore dans le flux de gaz vecteur quittant le récipient de réaction, et le dispositif de traitement ultérieur est réalisé de manière à pouvoir régler ultérieurement, de façon manuelle, une durée d'intégration, pour ainsi effectuer l'intégration de chacune des valeurs de concentration de l'oxygène et du dioxyde de carbone et/ou de l'azote et/ou du phosphore, telles qu'elles ont été enregistrées au cours du temps.
